# EUROPEAN PATENT SPECIFICATION

(11) **EP 0 535 470 B1**
(45) Date of publication and mention of the grant of the patent: **19.11.1997**
(21) Application number: 92116013.1
(22) Date of filing: 18.09.1992
(51) Int. Cl.: C07D 417/12, C07D 417/14, C10M 135/36

(54) **Succinimide derivatives of 2,5-dimercapto-1,3,4-thiadiazole**
Succinimidderivate von 2,5-Dimercapto-1,3,4-thiadiazol
Dérivés de succineimide de 2,5-dimércapto-1,3,4-thiadiazole

(30) Priority: 02.10.1991 US 770000
(43) Date of publication of application: 07.04.1993
(73) Proprietor: R.T. VANDERBILT COMPANY, Inc., Norwalk, Connecticut 06856-5150 (US)
(72) Inventor: Karol, Thomas J., Norwalk, Connecticut 06854 (US)
(74) Representative: Cohausz & Florack Patentanwälte

(56) References cited:
- EP-A- 218 816
- FR-A- 2 237 957

## Description

### BACKGROUND OF THE INVENTION

The present invention concerns succinimide derivatives of 2,5-dimercapto-1,3,4-thiadiazoles and their use as multifunctional additives for lubricating compositions.

Lubricating compositions ordinarily are formulated with various additives to enhance their performance. A problem sometimes encountered is that a particular additive has low solubility in oil or a combination of additives decreases the solubility of other additives in the lubricating composition resulting in undesirable haze or sediment formation. Therefore, it is desirable to use lubricating additives that can perform different functions.

The use of multifunctional lubricating additives containing the 2,5-dimercapto-1,3,4-thiadiazole group is known. 2,5-Dimercapto-1,3,4-thiadiazole is commonly called DMTD. Many DMTD derivatives have poor oil solubility and are incorporated into lubricating compositions with the aid of dispersants. However, dispersants employed in crankcase lubricating oils can degrade the elastomeric seals used in internal combustion engines. The dispersants can decrease the flexibility of the seals and increase their hardness causing crack formation.

Prior art dispersants include various nitrogen-bridged dispersants, including alkenylsuccinimide type. British Pat. No. 1,462,287 describes, among others, various succinimide dispersants which may be used to disperse dimercaptothiadiazoles having copper inhibiting activity. Specifically, a dispersant and 2,5-dimercapto-1,3,4-thiadiazole is interacted in amounts substantially greater than the stoichiometric amount necessary for salt formation. The precise chemical nature of the obtained homogeneous blends is not given. This intermediate blend can be reacted with a carboxylic acid to form a product of uncertain structure as described in U.S. Pat. No. 4,140,643.

Surprisingly, it has been discovered that certain amine dispersant derivatives of 2-(2-mercapto-1,3,4-thiadiazole-5-thio)succinic anhydride retain dispersant properties as well as display antiwear and antioxidant properties. The novel additives are compatible with elastomeric seals used in engines.

### SUMMARY OF THE INVENTION

In accordance with the invention, there are provided novel 1,3,4-thiadiazole compounds characterized by the structural formula 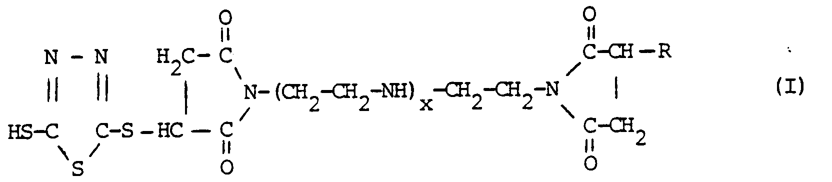 wherein x = 0 to 4, and R represents an alkenyl radical having fro 8 to 400 carbon atoms.

Another aspect of the invention concerns improved oil-based lubricating compositions comprising a major amount of base oil and an effective amount to impart antiwear and antioxidant properties to said compositions, of a 1,3,4-thiadiazole characterized by formula I.

A further aspect of the invention concerns a method for protection of engine parts from wear by applying improved lubricating oil compositions, the improvement of which consists of adding to the compositions an effective amount of a 1,3,4-thiadiazole characterized by the structural formula I.

### DESCRIPTION OF SPECIFIC EMBODIMENTS

The novel compounds of the invention may be prepared by reacting DMTD with maleic anhydride to yield the intermediate of the structural formula 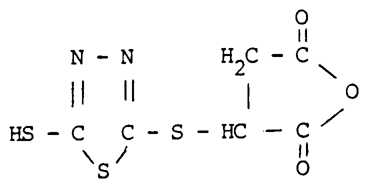 or its isomer. The intermediate is then reacted with N-polyethylene amine-substituted succinimide to yield compounds of formula I.

The N-polyamine-substituted succinimide reactants having dispersant properties are available commercially. A general preparation method is disclosed in U.S. Pat. No. 3,287,271. The dispersants are derived from alkenylsuccinic anhydride wherein the alkenyl group contains 8 to 400 carbon atoms and preferably from about 50 to 200 carbon atoms.

Particularly preferred are alkenyl groups derived from polyisobutene. The alkenyl radical is readily obtained by polymerizing olefins having 2 to 5 carbons, such as ethylene, propylene, isobutylene, pentene and similar alkenes.

The amine radical is derived from a polyamine of the formula H₂N(CH₂ - CH₂ - NH)ₓ - CH₂ - CH₂ - NH₂. Preferred compounds are wherein x is an integer from 0 to 4.

The thiadiazole derivatives of the invention are useful as additives for industrial lubricating compositions and engine oil formulations, as for example two cycle oils used in internal combustion engines.

The thiadiazole compounds possess multifunctional properties. They perform antiwear and oxidation inhibiting functions and are effective dispersants in lubricants and in engine oils. The dispersant property of the compounds facilitates their incorporation into the oil compositions and contribute to the overall stability and performance of the oil composition.

The lubricating compositions contemplated herein include lubricating oils, engine oils and lubricating greases containing a major amount of base oil. The base oil say be selected from naphthenic, aromatic, paraffinic, mineral, vegetable and synthetic oils. The synthetic oils may be selected from, among others, alkylene polymers, polysiloxanes and carboxylic acid esters.

The amount of the thiadiazole additive required to be effective for imparting antiwear and antioxidant characteristics to lubricating compositions may range from about 0.01 to 15.0 percent of the additive based on the lubricating composition.

The lubricating compositions may contain the necessary ingredients to formulate the compositions, as for example emulsifiers, other dispersants and viscosity improvers. Greases may be prepared by adding thickeners, as for example, salts and complexes of fatty acids, polyurea compounds, clays and quaternary ammonium bentonite complexes. Depending on the intended use of the lubricant, other functional additives may be added to enhance a particular property of the lubricant. The lubricating compositions may further contain extreme pressure agents, metal passivators, rust inhibitors, dispersants and other known antioxidants and antiwear agents.

The following examples are given for the purpose of further illustrating the invention. All percentages and parts are based on weight unless otherwise indicated.

### EXAMPLE 1

A reactor was charged with DMTD, 312.84 g, and dimethyl ketone, 722 g. Maleic anhydride, 196 g, was dissolved in dimethyl ketone, 300 g, and added to the reaction with cooling. The reaction was stirred for 30 min. and warned to 50° C. The product was recovered by filtering.

The intermediate product, 38 g, and N-triethylenetetraamine polyisobutenylmonosuccinimide, mol. wt. 1522, were reacted at 135-140° C for 2 hours and filtered.

### EXAMPLE 2

### Modified Falex Wear Test

A laboratory test was conducted by using the original Falex machine to simulate the valve train wear of an automobile engine. The V-blocks and pin were washed in mineral spirits with an ultrasonic cleaner, rinsed with acetone, air-dried and weighed. The test sample ( 60 g) was placed into the oil cup. The motor was switched on and the loading arm was placed on the ratchet wheel. Upon reaching the reference load of 227 kg, the ratchet wheel was disengaged and the load was maintained constant for 3.5 hours. Thereafter, the motor was switched off. The V-blocks and pin were washed, dried and weighed. The weight loss, a measure of wear, was recorded and compiled in Table I.

The test samples were prepared by incorporating the compounds of the invention in Motor Oil SAE 30, SF in the amount given in Table I. The oil was fully formulated with the exception of the antioxidant additive and contained 0.11 percent phosphorus. The results indicate that the present compounds afford good antiwear properties.

**TABLE I**

| Modified Falex Wear Test | | | |
|---|---|---|---|
| Sample | Active Ingredient | Percent | Total Weight Loss, mg |
| 1 | None | ― | 57.2 |
| 2 | Compound of Example 1 | 5.0 | 22.1 |

### EXAMPLE 3

### Thin Film Oxygen Uptake Test

The test was conducted essentially according to the method described by Chia-Soon et al, J. Am. Soc. Lubricating Eng., 40, 2 75-83, 1984. The oxidation induction time of the lubricant was measured under conditions simulating the high temperature oxidation processes in automotive engines by a modified rotary bomb oxidation test method ASTM D-2272. The test was conducted with 1.5 gram samples of SAE 30, SF motor oil. The oil was fully formulated with the exception of the antioxidant additive. A compound of the invention was added to the oil in the amount indicated in Table II. The test was conducted at 160° C and initial oxygen pressure of 620.6 kPa (90 psi). A "pass" oil has a high induction time, while a "fail" oil has a low time. The additive of the invention has good antioxidant properties as indicated by the data compiled in Table II.

**TABLE II**

| Thin Film Oxygen Uptake Test | | | |
|---|---|---|---|
| Sample | Active Ingredient | Percent | Average Induction Time, min. |
| 3 | None | --- | 108.0 |
| 4 | Compound of Example 1 | 5.0 | 162.5 |

The above embodiments have shown various aspects of the present invention.

## Claims

1. A thiadiazole compound selected from the group of compounds having the structural formula 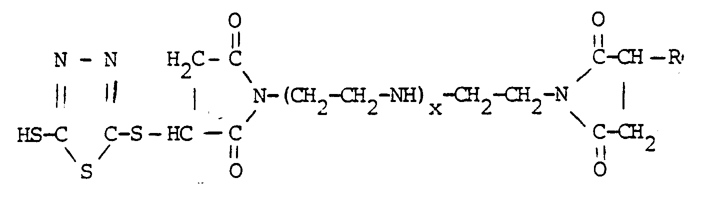 wherein x = 0 to 4, and R represents an alkenyl radical having from 8 to 400 carbon atoms.

2. A compound according to claim 1 wherein the alkenyl radical is polyisobutene.

3. A lubricating composition comprising a major portion of natural or synthetic oil of lubricating viscosity and a minor antiwear and oxidation inhibiting amount of a compound selected from the group consisting of compounds having the structural formula 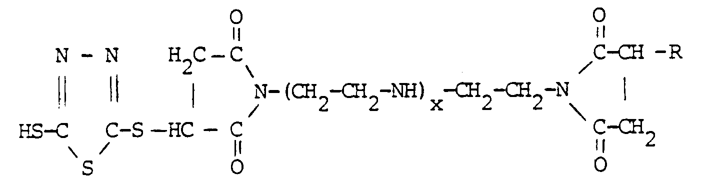 wherein x = 0 to 4, and R represents an alkenyl radical having from 8 to 400 carbon atoms.

4. A method for protecting engine parts from wear and deterioration which comprises contacting the surfaces with a lubricating composition comprising a major amount of base oil of lubricating viscosity, the improvement of which consists of adding to the oil a minor antiwear and oxidation inhibiting amount of an additive selected from the group of compounds having the structural formula 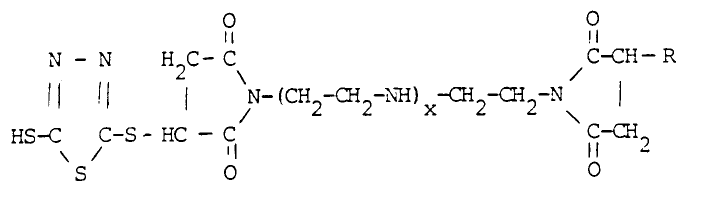 wherein x = 0 to 4 and R represents an alkenyl radical having from 8 to 400 carbon atoms and dispering additive into the base oil.

## Patentansprüche

1. Thiadiazol-Verbindung, ausgewählt aus der Gruppe der Verbindungen der Strukturformel 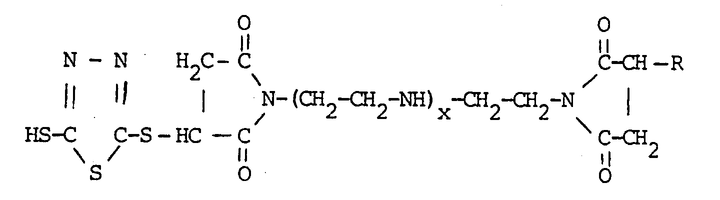 worin x = 0 bis 4 ist und R einen Alkenylrest mit 8 bis 400 Kohlenstoffatomen bedeutet.

2. Verbindung nach Anspruch 1, worin der Alkenylrest Polyisobuten ist.

3. Schmiermittel, enthaltend einen Hauptanteil eines natürlichen oder synthetischen Öls mit Schmierviskosität und eine geringe, den Verschleiß und die Oxidation hemmende Menge einer Verbindung, die ausgewählt ist aus der Gruppe der Verbindungen mit der Strukturformel 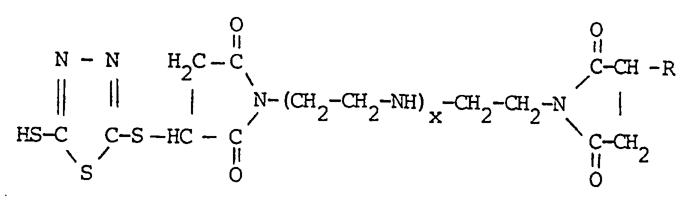 worin x = 0 bis 4 ist und R einen Alkenylrest mit 8 bis 400 Kohlenstoffatomen bedeutet.

4. Verfahren zum Schutz von Maschinenteilen vor Verschleiß und Zerstörung, welches das Inkontaktbringen der Oberflächen mit einem Schmiermittel umfaßt, welches einen Hauptanteil eines Grundöls mit Schmierviskosität enthält, wobei die Verbesserung darin besteht, daß dem Öl eine geringe, den Verschleiß und die Oxidation hemmende Menge einer Verbindung zugesetzt ist, die ausgewählt ist aus der Gruppe der Verbindungen mit der Strukturformel 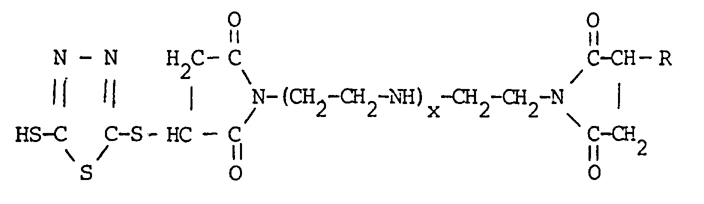 worin x = 0 bis 4 ist und R einen Alkenylrest mit 8 bis 400 Kohlenstoffatomen bedeutet und das Additiv in dem Grundöl dispergiert wird.

## Revendications

1. Composé de thidiazole sélectionné parmi le groupe de composés ayant la formule de structure 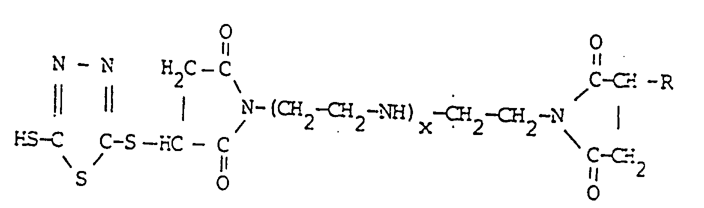 dans lequel x=0 à 4, et R représente un radical alkényle ayant de 8 à 400 atomes de carbone.

2. Composé selon la revendication 1 dans lequel le radical alkényle est le radical isobutylène.

3. Composition lubrifiante comprenant une portion majeure d'huile synthétique ou naturelle de viscosité lubrifiante et une quantité mineure de composé anti-usure ou inhibiteur d<oxydation sélectionné dans le groupe consistant des composés ayant la formule de structure 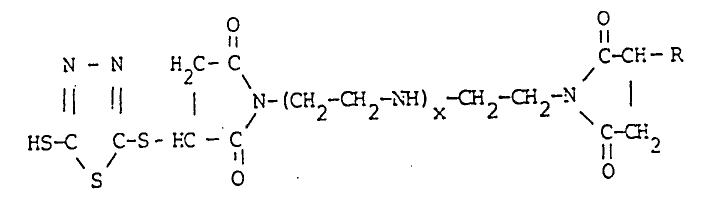 dans lequel x= 0 à 4, et R représente un radical alkényle ayant de 8 à 400 atomes de carbone.

4. Procédé pour protéger les pièces d'un moteur contre l'usure et la détérioration, comprenant la mise en contact des surfaces avec une composition lubrifiante contenant une quantité majeure d'une huile de base de viscosité lubrifiante, l'amélioration consistant en l'addition à l'huile d'une quantité mineure apte à résulter en des propriétés anti-usure et inhibitrices d'oxydation d'un additif sélectionné parmi le groupe de composés ayant la formule de structure suivante 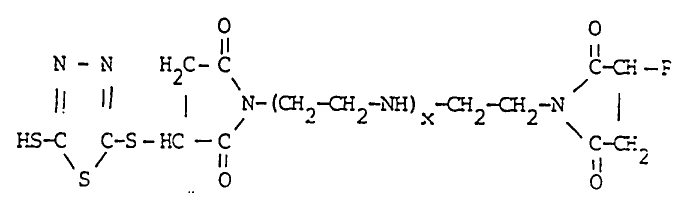 dans laquelle x= 0 à 4 et R représente un radical alkényle ayant de 8 à 400 atomes de carbone, et comprenant la mise en dispersion de l'additif dans l'huile de base.
